(19) Europäisches Patentamt
European Patent Office
Office européen des brevets

(11) Veröffentlichungsnummer: **0 077 298**
**B1**

# (12) EUROPÄISCHE PATENTSCHRIFT

(45) Veröffentlichungstag der Patentschrift: **19.06.85**

(21) Anmeldenummer: **82810412.5**

(22) Anmeldetag: **06.10.82**

(51) Int. Cl.⁴: **C 07 C 87/14**, C 07 C 87/28, C 07 C 87/34, C 07 D 211/26, C 07 D 307/14 // C07D225/02, C07D225/06, C07D401/04, C07D405/04, C07D471/10

(54) **Verfahren zur Herstellung von substituierten 1,11-Diaminoundecanen.**

(30) Priorität: **12.10.81 CH 6507/81**

(43) Veröffentlichungstag der Anmeldung: **20.04.83 Patentblatt 83/16**

(45) Bekanntmachung des Hinweises auf die Patenterteilung: **19.06.85 Patentblatt 85/25**

(84) Benannte Vertragsstaaten: **CH DE FR GB IT LI NL**

(56) Entgegenhaltungen:
**EP-A-0 011 599**
**DE-A-2 549 403**
**DE-A-2 831 353**

(73) Patentinhaber: **CIBA-GEIGY AG, Postfach, CH-4002 Basel (CH)**

(72) Erfinder: **Baumeister, Peter, St. Annaweg 24, CH-4113 Flüh (CH)**
Erfinder: **Reinehr, Dieter, Dr., Wolfsheule 10, D-7842 Kandern (DE)**
Erfinder: **Rosenegger, Eckehard, Oberdorfstrasse 53, CH-4125 Riehen (CH)**

**EP 0 077 298 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein neues Verfahren zur Herstellung von substituierten 1,11-Diaminoundecanen.

Gemäß EP Veröffentlichung Nr. 11 599 können in 1,10-Stellung substituierte 1,11-Diaminoundecane unter anderem dadurch hergestellt werden, daß man entsprechende 1-Aza-1,5,9-cyclododecatriene oder -dodecene mit einer unter den Reaktionsbedingungen nicht oxidierenden Säure, wie HCl oder Schwefelsäure, behandelt und anschließend in Gegenwart von flüssigem Ammoniak katalytisch hydriert.

Aus den deutschen Offenlegungsschriften 2 048 750 und 2 824 423 ist ferner bekannt, daß man primäre Alkylamine oder aliphatische oder cycloaliphatische Diamine dadurch erhalten kann, daß man in einer ersten Stufe entsprechende Aldehyde bzw. Dialdehyde mit Ammoniak bzw. Monoaminen zu Schiffbasen umsetzt und diese in einer zweiten Reaktionsstufe bei erhöhten Temperaturen und erhöhtem Druck mit Ammoniak und Wasserstoff in Gegenwart von Hydrierungskatalysatoren in die Amine bzw. Diamine überführt.

Es wurde nun gefunden, daß an sich sehr stabile 1-Aza-1,5,9-cyclododecatriene der in der deutschen Offenlegungsschrift 2 831 353 und der obengenannten EP Veröffentlichung beschriebenen oder ähnlichen Art überraschenderweise direkt, d. h. ohne Zusatz von Säure, in substituierte 1,11-Diaminoundecane übergeführt werden können. Damit entfällt auch die vom ökologischen Standpunkt aus unerwünschte Belastung der Abwässer mit Salzen.

Gegenstand der Erfindung ist somit ein neues Verfahren zur Herstellung von Verbindungen der Formel I

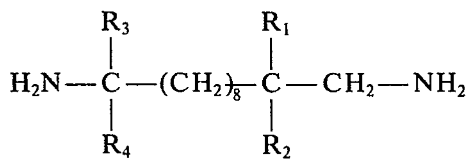

(I)

worin

| | |
|---|---|
| $R_1$ | $C_{1-12}$-Alkyl, |
| $R_2$ | Wasserstoff oder $C_{1-12}$-Alkyl, |
| $R_3$ | $C_{1-12}$-Alkyl, Cycloalkyl mit 4—12 Ring-C-Atomen, gegebenenfalls substituiertes Phenyl, Piperidyl, Tetrahydrofuryl oder Tetrahydrothienyl, |
| $R_4$ | Wasserstoff, $C_{1-12}$-Alkyl, Cycloalkyl mit 4—12 Ring-C-Atomen oder gegebenenfalls substituiertes Phenyl oder |

$R_1$ und $R_2$ und/oder $R_3$ und $R_4$ zusammen Alkylen mit 3—11 C-Atomen oder

$$—CH—C(CH_3)_2—NH—C(CH_3)_2—CH—$$

darstellen, indem man eine Verbindung der Formel II

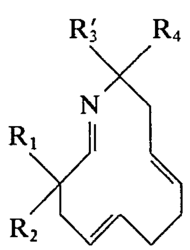

(II)

worin $R_1$, $R_2$ und $R_4$ die unter Formel I angegebene Bedeutung haben und $R_3'$ $C_{1-12}$-Alkyl, Cycloalkyl mit 4—12 Ring-C-Atomen, gegebenenfalls substituiertes Phenyl, Pyridyl, Furyl oder Thienyl oder zusammen mit $R_4$ Alkylen mit 3—11 C-Atomen oder

$$—CH—C(CH_3)_2—NH—C(CH_3)_2—CH—$$

darstellt, mit Ammoniak und Wasserstoff in Gegenwart eines Hydrierungskatalysators zu einer Verbindung der Formel I umsetzt.

Durch $R_1$ bis $R_4$ dargestellte Alkylgruppen können geradkettig oder verzweigt sein. Alkylgruppen $R_1$, $R_2$ und $R_4$ weisen bevorzugt 1—5 C-Atome auf und sind geradkettig. Alkylgruppen $R_3$ bzw. $R_3'$ weisen mit Vorteil 1—7 C-Atome auf; besonders bevorzugt sind Alkylgruppen $R_3$ und $R_3'$ mit 1—3 C-Atomen. Beispiele von Alkylgruppen $R_1$ bis $R_4$ sind: die Methyl-, Äthyl-, n-Propyl-, Isopropyl-, n-, sek- und tert-Butyl-, n-Pentyl-, 2- oder 3-Pentyl-, n-Hexyl-, 2- oder 3-Heptyl-, n-Octyl-, n-Decyl- und n-Dodecylgruppe.

Cycloalkylgruppen $R_3$, $R_3'$ und $R_4$ können unsubstituiert oder durch $C_{1-4}$-Alkylgruppen substituiert sein. Insbesondere handelt es sich dabei um durch eine Methyl- oder Äthylgruppe substituiertes Cycloalkyl. Bevorzugt sind Cycloalkylgruppen $R_3$, $R_3'$ und $R_4$ jedoch unsubstituiert und weisen 5—8 Ring-C-Atome auf. Besonders bevorzugt sind die Cyclopentyl- und vor allem die Cyclohexylgruppe.

Stellt $R_3$, $R_3'$ oder $R_4$ substituiertes Phenyl dar, so kommen als Substituenten vor allem Alkylgruppen mit 1—4 und besonders 1 oder 2 C-Atomen in Betracht. Phenylgruppen $R_3$, $R_3'$ oder $R_4$ können mehrere Alkylgruppen tragen, sind aber bevorzugt nur durch eine Alkylgruppe substituiert. Bevorzugt ist unsubstituiertes Phenyl.

Als Pyridyl-, Furyl- oder Thienylgruppen $R_3'$ kommen vor allem die Pyridyl-3-, Pyridyl-4-, Furyl-2- und Thienyl-2-gruppen in Betracht. Diese Reste $R_3'$ werden während der erfindungsgemäßen Umsetzung zu den entsprechenden Piperidyl-, Tetrahydrofuryl- und Tetrahydrothienylgruppen hydriert.

Durch $R_1$ und $R_2$ und/oder $R_3$ und $R_4$ zusammen dargestellte Alkylengruppen weisen bevorzugt 4—7 C-Atome auf. Insbesondere handelt es sich dabei um die Tetramethylen- und ganz besonders die Pentamethylgruppe.

Bevorzugt ist die Herstellung von Verbindungen der Formel I, worin $R_1$ Alkyl mit 1—5 C-Atomen, $R_2$ Wasserstoff oder Alkyl mit 1—5 C-Atomen, $R_3$ Alkyl mit 1—7 C-Atomen, Cycloalkyl mit 5—8 C-Atomen, besonders Cyclohexyl, Phenyl, Tetrahydrofuryl oder Piperidyl und $R_4$ Wasserstoff oder Alkyl mit 1—5 C-Atomen oder $R_3$ und $R_4$ zusammen Alkylen mit 4—7 C-Atomen oder

$$-CH-C(CH_3)_2-NH-C(CH_3)_2-CH-$$

darstellen.

Besonders bevorzugt ist die Herstellung von Verbindungen der Formel I, worin $R_1$ Alkyl mit 1—5 C-Atomen, $R_2$ Alkyl mit 1—5 C-Atomen oder Wasserstoff, $R_3$ Alkyl mit 1—5 C-Atomen, Phenyl, 2-Tetrahydrofuryl oder 3-Piperidyl und $R_4$ Wasserstoff oder Methyl oder $R_3$ und $R_4$ zusammen

$$-(CH_2)_4-,\ -(CH_2)_5-\ \text{oder}\ -CH-C(CH_3)_2-NH-C(CH_3)_2-CH-$$

darstellen. Ganz besonders bevorzugt ist die Herstellung von Verbindungen der Formel I, worin $R_1$ Methyl oder Äthyl, $R_2$ Wasserstoff, Methyl oder Äthyl, $R_3$ Alkyl mit 1—3 C-Atomen, besonders Isopropyl, oder Phenyl und $R_4$ Wasserstoff oder Methyl oder $R_3$ und $R_4$ zusammen $-(CH_2)_5-$ bedeuten.

Für die erfindungsgemäße Umsetzung können wasserfreier Ammoniak, wäßrige oder wäßrigorganische Ammoniaklösungen verwendet werden. Geeignete inerte organische Lösungsmittel sind z. B. Alkohole mit bis zu 6 C-Atomen, wie Methanol, Äthanol, Isopropanol, Butanole, Pentanole und Hexanole; Äther, vor allem cyclische, wie Tetrahydrofuran und Dioxan; ferner Kohlenwasserstoffe, z. B. Cyclohexan.

Bevorzugt verwendet man wäßrige Ammoniaklösungen, besonders 20—30%ige wäßrige Ammoniaklösungen.

Als Hydrierungskatalysatoren können an sich bekannte Verbindungen eingesetzt werden, wie Platin, Rhodium-, Palladium-, Ruthenium-, Nickel-, Kobalt- und Eisen-Katalysatoren. Bevorzugt sind Ruthenium-und Nickelkatalysatoren, besonders Raney-Nickel, Nickel auf Kieselgur und Ruthenium auf Kohle. Ganz besonders bevorzugt ist Ruthenium auf Kohle.

Die Katalysatoren werden zweckmäßig in Mengen von 0,1 bis 50 Gew.-%, bevorzugt 0,5 bis 25 Gew.-%, bezogen auf die Verbindung der Formel II, eingesetzt.

Die Reaktionstemperaturen liegen im allgemeinen zwischen 20 und 300° C, bevorzugt zwischen 100 und 250° C. Der Wasserstoffdruck liegt mit Vorteil zwischen 10 und 400 bar, besonders 20 und 100 bar.

Die Verbindungen der Formel II und der Ammoniak werden zweckmäßig in einem Mol-Verhältnis von 1 : 1 bis 1 : 50, vorzugsweise 1 : 1 bis 1 : 20, eingesetzt. Die Verbindungen der Formel II sind bekannt oder können nach dem in der deutschen Offenlegungsschrift 2 831 353 beschriebenen Verfahren hergestellt werden.

Nach Beendigung der Umsetzung können die Verbindungen der Formel I auf übliche Weise gereinigt werden, z. B. mittels Destillation.

Die Verbindungen der Formel I finden Anwendung als Härter für Epoxidharze oder aber zur Herstellung von transparenten Polyamiden; vgl. z. B. EP Veröffentlichung Nr. 11 599.

Beispiel 1

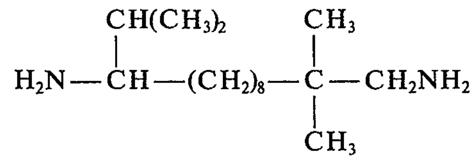

830 g einer 25%igen wäßrigen Ammoniaklösung (ca. 12,2 Mol $NH_3$) werden zusammen mit 80 g Raney-Nickel in einem 2,5-Liter-Magnetrührautoklaven vorgelegt. Nach dem Aufheizen des Autoklaven auf 180° C wird Wasserstoff bis zu einem Gesamtdruck von 100 bar aufgepreßt. Innerhalb einer Stunde werden mit einer Dosierpumpe 208 g (0,89 Mol) 3,3-Dimethyl-12-isopropyl-1-aza-1,5,9-cyclododecatrien eindosiert (Verhältnis 1-Aza-1,5,9-cyclododecatrien zu Ammoniak = 1 : 13,7). Nach einer Reaktionszeit von 9 Stunden kühlt man ab und destilliert. Nach einem Vorlauf von 10,2 g erhält man 199 g (0,776 Mol) 1-Isopropyl-10,10-dimethyl-1,11-diaminoundecan, entsprechend einer Ausbeute von 87% d. Th., Sdp. 87—89° C/4 Pa.

Beispiel 2

Verwendet man in Beispiel 1 bei sonst gleichen Reaktionsbedingungen anstelle von 80 g Raney-Nickel 80 g Nickel auf Kieselgur (55% Ni), so erhält man das 1-Isopropyl-10,10-dimethyl-1,11-diaminoundecan in einer Ausbeute von 88,5% d. Th.

Beispiel 3

Es wird wie in Beispiel 1 beschrieben vorgegangen, jedoch unter Verwendung von 52,2 g (0,225 Mol) 3,3-Dimethyl-12-isopropyl-1-aza-1,5,9-cyclododecatrien, 20 g Raney-Nickel und 75 g (1,1 Mol) einer 25%igen wäßrigen Ammoniaklösung (Verhältnis 1-Aza-1,5,9-cyclododecatrien zu Ammoniak = 1 : 4,9). Nach einer Reaktionszeit von 9 Stunden bei 175° C und einem Wasserstoffdruck von 100 bar erhält man bei der anschließenden Destillation 49 g (0,192 Mol) 1-Isopropyl-10,10-dimethyl-1,11-diaminoundecan; Ausbeute 85% d. Th.

Beispiel 4

Es wird wie in Beispiel 1 beschrieben vorgegangen, jedoch unter Verwendung von 26,1 g (0,112 Mol) 3,3-Dimethyl-12-isopropyl-1-aza-1,5,9-cyclododecatrien, 5 g Raney-Nickel und 105 g (1,55 Mol) einer 25%igen wäßrigen Ammoniaklösung. Nach einer Reaktionszeit von 9 Stunden bei 195—200° C und einem Wasserstoffdruck von 100 bar erhält man nach der Destillation 25 g (0,0975 Mol) 1-Isopropyl-10,10-dimethyl-1,11-diaminoundecan; Ausbeute 87% d. Th.

Beispiel 5

Es wird wie in Beispiel 4 beschrieben vorgegangen, jedoch unter Reduzierung der Reaktionszeit von 9 auf 3,5 Stunden. Man erhält das 1-Isopropyl-10,10-dimethyl-1,11-diaminoundecan in einer Ausbeute von 86% d. Th.

Beispiel 6

Es wird wie in Beispiel 1 beschrieben vorgegangen, jedoch unter Verwendung von 197 g (0,9 Mol) 3,3-Dimethyl-12-äthyl-1-aza-1,5,9-cyclododecatrien anstelle von 208 g 3,3-Dimethyl-12-isopropyl-1-aza-1,5,9-cyclododecatrien. Nach der Destillation erhält man 194 g (0,8 Mol) 1-Äthyl-10,10-dimethyl-1,11-diaminoundecan; Ausbeute 89% d. Th.; Sdp. 85—87°/4 Pa.

Beispiel 7

Es wird wie in Beispiel 1 beschrieben vorgegangen, jedoch unter Verwendung von 228 g (0,9 Mol) 3-Methyl-12-phenyl-1-aza-1,5,9-cyclododecatrien anstelle von 208 g (0,89 Mol) 3,3-Dimethyl-12-isopropyl-1-aza-1,5,9-cyclododecatrien. Nach der Destillation erhält man 210 g (0,76 Mol) 1-Phenyl-10-methyl-1,11-diaminoundecan; Ausbeute 84,5% d. Th.; Sdp. 137–140° C/1 Pa.

Beispiele 8–26

75 g einer 25%igen wäßrigen Ammoniaklösung werden zusammen mit 10 g Raney-Nickel und 20 ml 1normaler wäßriger NaOH sowie 52,2 g (0,2 Mol) 3,3-Dimethyl-12-isopropyl-1-aza-1,5,9-cyclododecatrien in einem Magnetrühr-Autoklaven vorgelegt. Nach dem Aufheizen der Reaktionsmischung auf 175° C wird Wasserstoffgas bis zu einem Gesamtdruck von 50 bar aufgepreßt. Nach einer Reaktionszeit von 9 Stunden kühlt man ab und destilliert. Man erhält 65% der theoretischen Ausbeute an 1-Isopropyl-10,10-dimethyl-1,11-diaminoundecan.

Auf analoge Weise wird 1-Isopropyl-10,10-dimethyl-1,11-diaminoundecan unter den in der folgenden Tabelle I angegebenen Reaktionsbedingungen hergestellt.

Tabelle I

| Beispiel Nr. | Katalysator | Zusatz | Zeit | Temp. °C | Druck | Ausbeute % d. Th. |
|---|---|---|---|---|---|---|
| 9 | 5 g Ru/C 5% | 20 ml 1N NaOH | 2,5 h | 180 | 50 bar | 95 |
| 10 | 20 g Ra-Ni | – | 17 h | 180 | 50 bar | 97 |
| 11 | 10 g Ra-Ni | – | 48 h | 180 | 100 bar | 90 |
| 12 | 10 g Ra-Ni | – | 6 h | 180 | 50 bar | 10 |
| 13 | 10 g Ra-Ni | – | 23 h | 180 | 50 bar | 80 |
| 14 | 10 g Ra-Ni | 2 ml 1N NaOH | 10,5 h | 180 | 50 bar | 60 |
| 15 | 2,5 g Ru/C 10% | 2 ml 1N NaOH | 0,75 h | 180 | 50 bar | 95 |
| 16 | 1,3 g Pd/C 5% | 2 ml 1N NaOH | 44 h | 180 | 50 bar | 15 |
| 17 | 10 g Ra-Ni | 1,6 g NaOAc | 28 h | 180 | 50 bar | 70 |
| 18 | 10 g Ra-Ni | – | 15 h | 200 | 50 bar | 60 |
| 19 | 10 g Ra-Ni | – | 42 h | 180 | 35 bar | 80 |
| 20 | 10 g Ra-Ni | 2 g $NEt_3$ | 22 h | 180 | 50 bar | 70 |
| 21 | 10 g Ra-Co | – | 43 h | 180 | 100 bar | 40 |
| 22 | 10 g Ra-Fe | – | 70 h | 200 | 100 bar | 20 |
| 23a | 1,2 g Ru/C 10% | 2 ml 1N NaOH | 14,5 h | 125–30 | 50 bar | 90 |
| 23b | | | 10 h | 125–30 | 20 bar | 90 |
| 24 | 10 g Ra-Cu | – | 54 | 175–180 | 50 bar | 40 |
| 25 | 5 g Pt/C 5% | – | 0,5 h | 175–180 | 50 bar | 10 |
| 26 | 5 g Rh/ $Al_2O_3$ 5% | – | 23 h | 175–180 | 50 bar | 30 |

## Beispiele 27–32

In einem Magnetrührautoklaven werden je 0,1 Mol einer Verbindung der Formel II, die zu einer Verbindung der Formel I mit den in Tabelle II angegebenen Bedeutungen von $R_1$ bis $R_4$ führt, zusammen mit 42,5 g einer 25%igen wäßrigen Ammoniaklösung und 1,25 g eines Ruthenium-auf-Aktivkohle-Hydrierkatalysators vorgelegt (10% Ru). Nach dem Aufheizen der Reaktionsmischung wird Wasserstoffgas bis zu einem Gesamtdruck von 50 bar aufgepreßt.

Findet keine Druckabnahme mehr statt, wird abgekühlt und das Diamin durch Destillation abgetrennt. Die Reaktionszeiten und Ergebnisse sind in Tabelle II zusammengefaßt.

Tabelle II

| Beispiel Nr. | Diamin der Formel I | | | | Reaktionszeit | $n_D^{20}$ | Ausbeute % d. Th. |
|---|---|---|---|---|---|---|---|
| | $R_1$ | $R_2$ | $R_3$ | $R_4$ | | | |
| 27 | $CH_3$ | $CH_3$ | $C_2H_5$ | H | 2,5 h | 1,4622 | 75 |
| 28 | $CH_3$ | H | $CH_3$ | $CH_3$ | 28 h | 1,4585 | 40 |
| 29 | $CH_3$ | $CH_3$ | 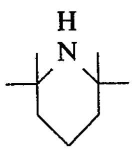 | *) | 19,5 h | 1,4887 | 75 |
| 30 | $CH_3$ | H | $C_6H_5$ | H | 22 h | 1,5095 | 60 |
| 31 | $C_2H_5$ | $C_2H_5$ | $iC_3H_7$ | H | 16 h | 1,4704 | 50 |
| 32 | $C_2H_5$ | H | $-(CH_2)_5-$ | | 6 h | 1,4815 | 80 |

*) Das entsprechende Ausgangsprodukt der Formel II kann wie folgt hergestellt werden: In einem 2,5-Liter-Sulfierkolben werden 400 g 2-Methylallylamin in 400 ml Toluol gelöst. Die Lösung wird mit 450 g Molekularsieb versetzt. Anschließend tropft man eine Lösung von 700 g 2,2,6,6-Tetramethyl-4-piperidon in 300 ml Toluol so zu, daß die Reaktionstemperatur bei 30–35° C gehalten wird. Nach vollständiger Zugabe wird noch 6 Stunden bei Raumtemperatur gerührt. Es wird vom Molekularsieb abfiltriert, und das Lösungsmittel wird am Rotationsverdampfer entfernt. Die Destillation des Rückstandes gibt 778 g N-(2,2,6,6-Tetramethyl-4-piperidyliden)-2-methylallylamin, entsprechend 83% d. Th., als farblose Flüssigkeit vom Kp. 63° C bei 40 Pa.

In einem 500-ml-Dreihalskolben mit Rückflußkühler werden 162,5 g des N-(2,2,6,6-Tetramethyl-4-piperidyliden)-2-methylallylamins in 162 ml Toluol gelöst und mit 4,86 g Kalium-tert-butylat versetzt. Die Reaktionsmischung wird unter Rühren mit 50 ml Dimethylsulfoxid versetzt und 8 Stunden bei 50° C gerührt. Danach wird die organische Phase mit 100 ml dest. Wasser ausgeschüttelt und der organische Teil nach Abtrennen des Lösungsmittels destilliert. Man erhält 136,9 g N-(2,2,6,6-Tetramethyl-4-piperidyliden)-2-methyl-1-amino-1-propen als farblose Flüssigkeit vom Kp. 56° C bei 40 Pa (82% d. Th.).

136,0 g des N-(2,2,6,6-Tetramethyl-4-piperidyliden)-2-methyl-1-amino-1-propens, 3,9 g Nickelacetyl acetonat und 1,85 g Trimethylphosphit werden in einer 500-ml-Schlenk-Ampulle unter Argon in 150 ml Toluol gelöst und mit 5,5 ml Äthoxydiäthylaluminium versetzt. Anschließend werden bei 86 g 1,3-Butadien bei −78° C einkondensiert, und die Mischung wird unter Rühren auf Raumtemperatur erwärmt. Nach 1 Stunde wird auf 40° C aufgeheizt und 18 Stunden bei dieser Temperatur gerührt. Nach dem Abkühlen und Abtrennen des Lösungsmittels wird destilliert. Man erhält 106,8 g 12-Spiro-(2,2,6,6-tetramethyl-4-piperidyl)-3,3-dimethyl-1-aza-1,5,9-cyclododecatrien als farbloses Öl vom Kp. 136° C bei 40 Pa (51,6% d. Th.).

## Beispiel 33

Beispiel 27 wird wiederholt, jedoch unter Verwendung von 24,35 g (0,1 Mol) 12-(2-Furyl)-3-methyl-1-aza-1,5,9-cyclododecatrien. Nach der Destillation erhält man 80% d. Th. 1-(2-Tetrahydrofuryl)-10-methyl-1,11-diaminoundecan; $n_D^{20}$: 1,4800.

Beispiel 34

Beispiel 27 wird wiederholt, jedoch unter Verwendung von 25,4 g (0,1 Mol) 12-(3-Pyridyl)-3-methyl-1-aza-1,5,9-cyclododecatrien. Nach 33 Stunden Reaktionszeit erhält man nach der Aufarbeitung 45% d. Th. 1-(3-Piperidino)-10-methyl-1,11-diaminoundecan; $n_D^{20}$: 1,4762.

Beispiel 35

0,25 Mol 3,3-Dimethyl-12-isopropyl-1-aza-1,5,9-cyclododecatrien werden gemeinsam mit 85 ml 25%iger wäßriger Ammoniaklösung und 1,5 g Ruthenium auf Aktivkohle Hydrierkatalysator (10% Ru) vorgelegt und unter einem Wasserstoffdruck von 50 bar auf 180° C aufgeheizt. Nach einer Reaktionszeit von 30 Minuten ist keine weitere $H_2$-Aufnahme mehr festzustellen. Die destillative Aufarbeitung der filtrierten Reaktionsmasse ergibt das Produkt in einer Ausbeute von 90% der Theorie.

Anwendungsbeispiel

i) In einem Rundkolben, der mit Rührer, Rückflußkühler und Tropftrichter versehen ist, werden 0,1 Mol Terephthalsäure in 300 ml 70%igem Äthanol zum Sieden erhitzt. In die kochende Suspension läßt man unter Rühren 0,1 Mol 1-Isopropyl-10,10-dimethyl-1,11-diaminoundecan im Verlaufe von ca. 10 Minuten aus dem Tropftrichter einfließen und spült darin haftende Diaminreste mit etwas Äthanol quantitativ in das Reaktionsgemisch. Die dabei entstandene klare Lösung läßt man unter ständigem Rühren erkalten, filtriert das dabei ausgefallene Salz ab und trocknet es im Vakuum bei 90° C.

ii) In ein Bombenrohr, welches mit einem Schraubdeckel mit eingebautem Überdruckventil ausgestattet ist, werden folgende Komponenten eingewogen:

a) 4,4'-Diamino-3,3'-dimethyldicyclohexylmethan,
b) eine diesem Diamin äquivalente Menge Isophthalsäure,
c) 70 Gew.-% des gemäß i) erhaltenen Salzes, bezogen auf das Gesamtgewicht der Reaktionskomponenten.

Nachdem man die Luft im Bombenrohr vollständig durch Stickstoff oder ein anderes Inertgas verdrängt hat, verschließt man das Bombenrohr und taucht es in ein Salzbad, dessen Temperatur 270° C beträgt. Nach 30 bis 60 Minuten ist eine homogene, glasklare Schmelze entstanden. Nach insgesamt 3 Stunden wird die Vorkondensation abgebrochen, indem man das Bombenrohr aus dem Salzbad entfernt und den Überdruck durch Öffnen des Ventils abläßt. Das erstarrte glasklare Vorkondensat wird aus dem Bombenrohr entfernt und in ein Kondensationsgefäß übergeführt. Unter strengem Ausschluß von Luft und unter dauerndem Durchleiten von Stickstoff wird die Schmelze bei 280° C Salzbadtemperatur 5 Stunden polykondensiert, wobei das Reaktionswasser durch den Stickstoffstrom laufend entfernt wird. Beim Abkühlen erstarrt die Schmelze zu einer glasklaren Masse. 2—3 g des hergestellten Copolyamids werden in einer beheizbaren hydraulischen Presse bei 270° C zu einer Folie von ca. 0,4 bis 1 mm Dicke verpreßt. Das erhaltene Copolyamid hat eine reduzierte spezifische Viskosität $\eta$ red. von 1,09 dl/g, gemessen an einer 0,5%igen Lösung in m-Kresol bei 25° C, und eine Glasumwandlungstemperatur von 162° C (gemessen im Differentialkalorimeter, DSC). Das Copolyamid weist ferner eine sehr gute Kochwasserbeständigkeit auf, das heißt, auch nach mehreren Tagen ist keine Beeinträchtigung der Transparenz feststellbar.

**Patentansprüche**

1. Verfahren zur Herstellung von Verbindungen der Formel I

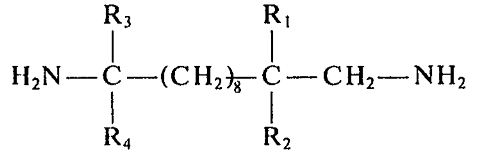

(I)

worin

$R_1$ $C_{1-12}$-Alkyl,
$R_2$ Wasserstoff oder $C_{1-12}$-Alkyl,

$R_3$ $C_{1-12}$-Alkyl, Cycloalkyl mit 4–12 Ring-C-Atomen, gegebenenfalls substituiertes Phenyl, Piperidyl, Tetrahydrofuryl oder Tetrahydrothienyl,
$R_4$ Wasserstoff, $C_{1-12}$-Alkyl, Cycloalkyl mit 4–12 Ring-C-Atomen oder gegebenenfalls substituiertes Phenyl oder $R_1$ und $R_2$ und/oder
$R_3$ und $R_4$ zusammen Alkylen mit 3–11 C-Atomen oder

$$—CH—C(CH_3)_2—NH—C(CH_3)_2—CH—$$

darstellen, dadurch gekennzeichnet, daß man eine Verbindung der Formel II

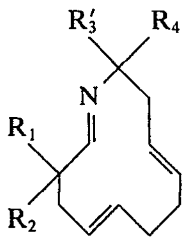

, (II)

worin $R_1$, $R_2$ und $R_4$ die unter Formel I angegebene Bedeutung haben und $R'_3$ $C_{1-12}$-Alkyl, Cycloalkyl mit 4–12 Ring-C-Atomen, gegebenenfalls substituiertes Phenyl, Pyridyl, Furyl oder Thienyl oder zusammen mit $R_4$ Alkylen mit 3–11 C-Atomen oder

$$—CH—C(CH_3)_2—NH—C(CH_3)_2—CH—$$

darstellt, mit Ammoniak und Wasserstoff in Gegenwart eines Hydrierungskatalysators zu einer Verbindung der Formel I umsetzt.

2. Verfahren nach Anspruch 1, dadurch gekennzeichnet, daß man eine Verbindung der Formel I herstellt, worin $R_1$ Alkyl mit 1–5 C-Atomen, $R_2$ Wasserstoff oder Alkyl mit 1–5 C-Atomen, $R_2$ Wasserstoff oder Alkyl mit 1–5 C-Atomen, $R_3$ Alkyl mit 1–7 C-Atomen, Cycloalkyl mit 5–8 C-Atomen, besonders Cyclohexyl, Phenyl, Tetrahydrofuryl oder Piperidyl und $R_4$ Wasserstoff oder Alkyl mit 1–5 C-Atomen oder $R_3$ und $R_4$ zusammen Alkylen mit 4–7 C-Atomen oder

$$—CH—C(CH_3)_2—NH—C(CH_3)_2—CH—$$

bedeuten.

3. Verfahren nach Anspruch 1, dadurch gekennzeichnet, daß man eine Verbindung der Formel I herstellt, worin $R_1$ Alkyl mit 1–5 C-Atomen, $R_2$ Alkyl mit 1–5 C-Atomen oder Wasserstoff, $R_3$ Alkyl mit 1–5 C-Atomen, Phenyl, 2-Tetrahydrofuryl oder 3-Piperidyl und $R_4$ Wasserstoff oder Methyl oder $R_3$ und $R_4$ zusammen

$—(CH_2)_4—$, $—(CH_2)_5—$ oder $—CH—C(CH_3)_2—NH—C(CH_3)_2—CH—$

bedeuten.

4. Verfahren nach Anspruch 1, dadurch gekennzeichnet, daß man eine Verbindung der Formel I herstellt, worin $R_1$ Methyl oder Äthyl, $R_2$ Wasserstoff, Methyl oder Äthyl, $R_3$ Alkyl mit 1–3 C-Atomen, besonders Isopropyl, oder Phenyl und $R_4$ Wasserstoff oder Methyl oder $R_3$ und $R_4$ zusammen

$$—(CH_2)_5—$$

bedeuten.

5. Verfahren nach Anspruch 1, dadurch gekennzeichnet, daß man die Umsetzung in wäßrigem Ammoniak vornimmt.

6. Verfahren nach Anspruch 1, dadurch gekennzeichnet, daß man als Katalysator Raney-Nickel, Nikkel auf Kieselgur und insbesondere Ruthenium auf Kohle verwendet.

7. Verfahren nach Anspruch 1, dadurch gekennzeichnet, daß man die Umsetzung bei einer Temperatur zwischen 100 und 250° C vornimmt.

8. Verfahren nach Anspruch 1, dadurch gekennzeichnet, daß man die Umsetzung bei einem Druck zwischen 20 und 100 bar vornimmt.

9. Verfahren nach Anspruch 1, dadurch gekennzeichnet, daß man den Katalysator in einer Menge von 0,5 bis 25 Gew.-%, bezogen auf die Verbindung der Formel II, einsetzt.

## Claims

1. A process for the preparation of a compound of the formula I

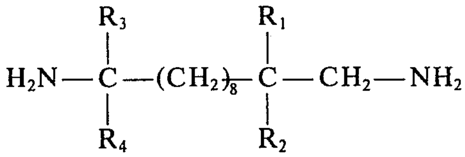

(I)

wherein

$R_1$ is $C_1$–$C_{12}$alkyl,
$R_2$ is hydrogen or $C_1$–$C_{12}$alkyl,
$R_3$ is $C_1$–$C_{12}$alkyl, cycloalkyl having 4 to 12 ring carbon atoms, or is unsubstituted or substituted phenyl, or is piperidyl, tetrahydrofuryl or tetrahydrothienyl,
$R_4$ is hydrogen, $C_1$–$C_{12}$alkyl, cycloalkyl having 4 to 12 ring carbon atoms, or is unsubstituted or substituted phenyl, or
$R_1$ and $R_2$ and/or $R_3$ and $R_4$ together are alkylene having 3 to 11 carbon atoms, or are

$$—CH—C(CH_3)_2—NH—C(CH_3)_2—CH—,$$

which process comprises reacting a compound of the formula II

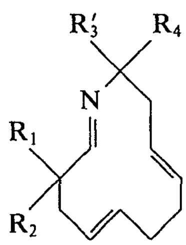

(II)

wherein $R_1$, $R_2$ and $R_4$ are as defined for formula I, and $R_3'$ is $C_1$–$C_{12}$alkyl, cycloalkyl having 4 to 12 ring carbon atoms, or unsubstituted or substituted phenyl, or is pyridyl, furyl or thienyl, or together with $R_4$ is alkylene having 3 to 11 carbon atoms or

$$—CH—C(CH_3)_2—NH—C(CH_3)_2—CH—,$$

with ammonia and hydrogen, in the presence of a hydrogenation catalyst.

2. A process according to claim 1 for the preparation of a compound of the formula I, wherein $R_1$ is alkyl having 1 to 5 carbon atoms, $R_2$ is hydrogen or alkyl having 1 to 5 carbon atoms, $R_3$ is alkyl having 1 to 7 carbon atoms, cycloalkyl having 5 to 8 carbon atoms, preferably cyclohexyl, phenyl, tetrahydrofuryl or piperidyl, and $R_4$ is hydrogen or alkyl having 1 to 5 carbon atoms, or $R_3$ and $R_4$ together are alkylene having 4 to 7 carbon atoms or

$$—CH—C(CH_3)_2—NH—C(CH_3)_2—CH.$$

3. A process according to claim 1 for the preparation of a compound of the formula I, wherein $R_1$ is alkyl having 1 to 5 carbon atoms, $R_2$ is alkyl having 1 to 5 carbon atoms or hydrogen, $R_3$ is alkyl having 1 to 5 carbon atoms, phenyl, 2-tetrahydrofuryl or 3-piperidyl, and $R_4$ is hydrogen or methyl, or $R_3$ and $R_4$ together are

$—(CH_2)_4—$, $—(CH_2)_5—$ or $—CH—C(CH_3)_2—NH—C(CH_3)_2—CH.$

4. A process according to claim 1 for the preparation of a compound of the formula I, wherein $R_1$ is methyl or ethyl, $R_2$ is hydrogen, methyl or ethyl, $R_3$ is alkyl having 1 to 3 carbon atoms, preferable isopropyl, or phenyl, and $R_4$ is hydrogen or methyl, or $R_3$ and $R_4$ together are

$$—(CH_2)_5—.$$

5. A process according to claim 1, wherein the reaction is performed in aqueous ammonia.

6. A process according to claim 1, wherein the catalyst used is Raney nickel, nickel on kieselguhr and, in particular, ruthenium on charcoal.

7. A process according to claim 1, wherein the reaction is performed at a temperature in the range

from 100° to 250°C.

8. A process according to claim 1, wherein the reaction is performed under a pressure in the range from 20 to 100 bar.

9. A process according to claim 1, wherein the catalyst is used in an amount of 0.5 to 25% by weight, based on the compound of formula II.

**Revendications**

1. Procédé de préparation de composés répondant à la formule I:

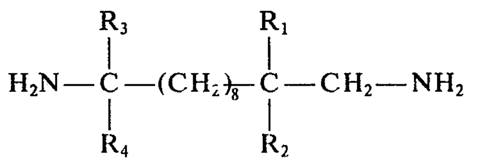

(I)

dans laquelle

$R_1$ représente un alkyle en $C_1$—$C_{12}$,
$R_2$ représente l'hydrogène ou un alkyle en $C_1$—$C_{12}$,
$R_3$ représente un alkyle en $C_1$—$C_{12}$, un cycloalkyle dont le noyau contient de 4 à 12 atomes de carbone, ou un radical phényle, pipéridyle, tétrahydrofuryle ou tétrahydrothiényle éventuellement substitué,
$R_4$ représente l'hydrogène, un alkyle en $C_1$—$C_{12}$, un cycloalkyle dont le noyau contient de 4 à 12 atomes de carbone, ou un radical phényle éventuellement substitué, ou encore
$R_1$ et $R_2$, et/ou $R_3$ et $R_4$, forment endemble un alkylène contenant de 3 à 11 atomes de carbone ou un radical

$$—CH_2—C(CH_3)_2—NH—C(CH_3)_2—CH_2— ,$$

procédé caractérisé en ce qu'on fait réagir un composé répondant à la formule générale II:

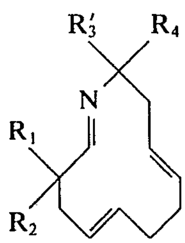

(II)

dans laquelle $R_1$, $R_2$ et $R_4$ ont les significations données au-dessous de la formule I et $R_3'$ représente un alkyle en $C_1$—$C_{12}$, un cycloalkyle dont le noyau contient de 4 à 12 atomes de carbone, ou un radical phényle, pyridyle, furyle ou thiényle éventuellement substitué, ou encore forme avec $R_4$ un alkylène en $C_3$—$C_{11}$ ou un radical

$$—CH_2—C(CH_3)_2—NH—C(CH_3)_2—CH_2— ,$$

avec l'ammoniac et l'hydrogène en présence d'un catalyseur d'hydrogénation, réaction qui conduit à un composé de formule I.

2. Procédé selon la revendication 1 caractérisé en ce qu'on prépare un composé de formule I dans lequel $R_1$ représente un alkyle en $C_1$—$C_5$, $R_2$ l'hydrogène ou un alkyle en $C_1$—$C_5$, $R_3$ un alkyle en $C_1$—$C_7$, un cycloalkyle en $C_5$—$C_8$, en particulier un cyclohexyle, un phényle, un tétrahydrofuryle ou un pipéridyle et $R_4$ l'hydrogène ou un alkyle en $C_1$—$C_5$, ou encore $R_3$ et $R_4$ forment ensemble un alkylène contenant de 4 à 7 atomes de carbone ou un radical

$$—CH_2—C(CH_3)_2—NH—C(CH_3)_2—CH_2— .$$

3. Procédé selon la revendication 1 caractérisé en ce qu'on prépare un composé de formule I dans lequel $R_1$ représente un alkyle en $C_1$—$C_5$, $R_2$ un alkyle en $C_1$—$C_5$ ou l'hydrogène, $R_3$ un alkyle en $C_1$—$C_5$, un phényle, un tétrahydrofuryle-2 ou un pipéridyle-3 et $R_4$ l'hydrogène ou un méthyle, ou encore $R_3$ et

$R_4$ forment ensemble un radical

$$-(CH_2)_4-,\ -(CH_2)_5-\ \text{ou}\ -CH_2-C(CH_3)_2-NH-C(CH_3)_2-CH_2-.$$

4. Procédé selon la revendication 1 caractérisé en ce qu'on prépare un composé de formule I dans lequel $R_1$ représente un méthyle ou un éthyle, $R_2$ l'hydrogène, un méthyle ou un éthyle, $R_3$ un alkyle en $C_1-C_3$, en particulier un isopropyle, ou un phényle et $R_4$ l'hydrogène ou un méthyle, ou encore $R_3$ et $R_4$ forment ensemble un radical

$$-(CH_2)_5-.$$

5. Procédé selon la revendication 1 caractérisé en ce qu'on effectue la réaction dans une solution ammoniacale aqueuse.

6. Procédé selon la revendication 1 caractérisé en ce qu'on utilise, comme catalyseur, dù nickel de Raney, du nickel sur kieselguhr ou, mieux, du ruthénium sur charbon.

7. Procédé selon la revendication 1 caractérisé en ce qu'on effectue la réaction à une température comprise en 100 et 250°C..

8. Procédé selon la revendication 1 caractérisé en ce qu'on effectue la réaction sous une pression comprise entre 20 et 100 bar.

9. Procédé selon la revendication 1 caractérisé en ce que le catalyseur est mis en jeu en une quantité de 0,5 à 25% en poids par rapport au composé de formule II.